# EUROPEAN PATENT APPLICATION

(11) **EP 2 859 946 A1**
(43) Date of publication of application: **15.04.2015**
(21) Application number: 13382404.5
(22) Date of filing: 11.10.2013
(51) Int. Cl.: B01J 37/08, B01J 38/00, B01J 38/02, B01J 38/52, B01J 38/56, B01J 38/60, B01J 21/06, B01J 21/08, C07D 301/19, B01J 21/20

(54) **Process for regenerating heterogeneous epoxidation catalysts and their use to catalyze epoxidation reactions**

(71) Applicant: Repsol, S.A., 28045 Madrid (ES)
(72) Inventor: VEGA BERMEJO, Luis, 28935 MÓSTOLES (ES); MASA LORENZO, María de la O, 28935 MÓSTOLES (ES); PEDROLA VIDAL, Jordi, 28934 MÓSTOLES (ES); GÓMEZ ALDA, David, 28935 MÓSTOLES (ES); SAN NICOLÁS SAYANS, Estanislao, 28935 MÓSTOLES (ES)
(74) Representative: ZBM Patents - Zea, Barlocci & Markvardsen

(57) **Abstract**

A process for regenerating a heterogeneous titanium-containing silicon oxide catalyst comprising the steps of contacting the heterogeneous titanium-containing silicon oxide catalyst to be regenerated with with a solvent which is inert with the catalyst active sites, washing with an acid aqueous solution, impregnating the washed catalyst thus obtained with a titanium-containing impregnating agent, and calcinating the impregnated catalyst; as well as a process for the preparation of an alkylene oxide comprising carrying out the previous regeneration process, followed by reacting an olefin with an organic hydroperoxide in the presence of the regenerated heterogeneous titanium-containing silicon oxide catalyst.

## Description

The present invention relates to a method of regenerating the activity and selectivity of a titanium-containing silicon oxide catalyst that has been used to catalyze an oxidation reaction such as the epoxidation of an olefin with an organic hydroperoxide.

### BACKGROUND ART

It is well known in the art to produce alkylene oxides, such as propylene oxide, by epoxidation of the corresponding olefin using active oxygen species such as hydrogen peroxide or an organic hydroperoxide as the source of oxygen. For instance, a commonly known method for manufacturing propylene oxide is the co-production of propylene oxide and styrene starting from ethylbenzene. In general such process involves the steps of (i) reacting ethylbenzene with oxygen or air to form ethylbenzene hydroperoxide, (ii) reacting the ethylbenzene hydroperoxide thus obtained with propene in the presence of an epoxidation catalyst to yield propylene oxide and 1-phenyl ethanol, and (iii) dehydrating the 1-phenyl ethanol into styrene using a suitable dehydration catalyst.

Titanium-containing heterogeneous epoxidation catalysts are known in the art. Examples of such catalysts are for instance described in US4367342 and EP345856. US4367342 discloses the use of inorganic oxygen compounds of silicon in chemical composition with at least 0.1 % by weight of an oxide or hydroxide of titanium, while EP345856 discloses a titania-on-silica heterogeneous catalyst which is obtainable by impregnating a silicon compound with a stream of gaseous titanium tetrachloride followed by calcination and hydrolysis steps and optionally a silylation step. Alternatively, heterogeneous titanium-containing catalysts can be prepared by a liquid-phase impregnation process as described in e.g. US6011162 among others.

Such titanium-containing silicon oxide heterogeneous catalysts tipically comprise an inorganic oxygen compound of silicon in chemical combination with an inorganic oxygen compound of titanium in a tetravalent oxidation state, which is preferablly combined with the oxygen compound of silicon. When these heterogeneous epoxidation catalysts are used in a continuous process such as a fixed bed reactor, the catalyst activity decreases with time until it must be replaced by fresh or regenerated catalyst. The used catalyst may either be disposed of or may be regenerated for reuse.

Reuse is highly economic interesting because of catalyst consumption reduction but it is also a measure to minimize solid waste generation.

In WO9828072 a regeneration process for this type of catalysts is disclosed, which comprises contacting the used catalyst with a particular solvent (water, alcohols, ketones, ethers, etc.) at a temperature of from 20 to 400 °C.

In US5798313 a regeneration method for titanium-containing heterogeneous olefin epoxidation catalysts is disclosed, wherein the used epoxidation catalyst is heated at a temperature of at least 700 °C in the presence of oxygen.

Nevertheless these methods don't load the used catalyst with active metal, and it has been observed that the regeneration of the catalysts following the above mentioned methods is temporary and the catalysts loses its regained activity very rapidly.

There are other inventions that convert the deactivated catalyst into a suitable support material which can be freshly loaded with catalytically active metal.

In EP1251960, a process for the preparation of a catalyst support material comprising the steps of: (a) subjecting a used titania-on-silica catalyst to a decoking treatment (300-700°C in the presence of oxygen), (b) washing the decoked catalyst with a washing liquid selected from an aqueous solution of a mineral acid, an aqueous solution of an ammonium salt and combinations thereof, and (c) drying and calcining (temperature less than 500°C) the washed and decoked catalyst to yield the catalyst support material. The support material thus obtained is suitably used as support material for titania in a heterogeneous catalyst for the epoxidation of olefins into alkylene oxides.

In EP1539346, used titanium-containing silicon oxide catalysts are regenerated by heating the used catalyst at a temperature of at least 400 °C in the presence of a oxygen-containing gas stream, followed by impregnation with a titanium source, and then calcining the impregnated catalyst to form a reactivated catalyst.

However, these methods are highly energy and time consuming and requires sophisticated reactor design and hardware requirements suitable for heating the catalyst bed to sucha high temperatures.

Accordingly, it is always desirable to develop new and improved methods of regeneration of used catalysts, which results in the restoration of their activity and selectivity.

### SUMMARY OF THE INVENTION

The present invention provides an effective regeneration process of a used titanium-containing silicon oxide catalyst, in order to restore its activity and selectivity, wherein such catalyst has been preferably used to catalyze epoxidation of an olefin using an organic hydroperoxide. The method comprising contacting the used catalyst with ethylbenzene at a temperature of from 20-80 °C during 3-6 hours, followed by acid aqueous treatment, impregnation with a titanium-containing compound, and then calcining the impregnated catalyst to form the regenerated catalyst.

Accordingly, the present invention relates to a method of regenerating a heterogeneous titanium-containing silicon oxide catalyst comprising the steps of:
a) contacting the heterogeneous titanium-containing silicon oxide catalyst to be regenerated with a solvent which is inert with the catalyst active sites, preferably ethylbenzene, at a temperature ranging from 20-90 °C for 2-6 hours
b) washing the catalyst obtained in step a) with an acid aqueous solution, preferably at a temperature ranging from 20 - 90 °C for 2 - 6 hours;
c) impregnating the washed catalyst thus obtained with a titanium-containing impregnating agent; and
d) calcinating the impregnated catalyst at a temperature ranging from 400 - 1000 °C to obtain a regenerated catalyst.

We surprisingly found that regeneration of used catalysts by the method of the present invention resulted in a substantial activity and selectivity improvement as compared to the used catalyst. A calcination, decoking or any high temperature treatment is avoided in the first stage of the regenerating method (prior to catalyst manufacturing) resulting a less energy consumption procedure.

Another aspect of the present invention is to provide a process for the preparation of an alkylene oxide, said process comprising reacting an olefin with an organic hydroperoxide in the presence of a regenerated heterogeneous titania-on-silica catalyst, wherein the catalyst has been regenerated in accordance with the process as described above.

### DETAILED DESCRIPTION OF THE INVENTION

The titanium-containing silicon oxide catalysts to be regenerated in accordance with the present invention are well known in the art. Such catalysts generally comprise silicon oxide in chemical combination with a titanium oxide or hydroxide.

The siliceous-based carrier, may be preferably an amorphous silica. In the context of the present invention, the term "amorphous silica" is used here for the pure forms of SiO₂ such as colloidal silica, precipitated silica, silica gel, pyrogenic silica, fumed silica, quartz glass, fused silica. Another class of inorganic siliceous-based carriers suitable for the preparation of the titanium-containing silicon oxide catalysts to be regenerated in accordance with the present invention are synthetic inorganic oxide materials containing a major proportion of silica. Such materials are known as refractory oxides and includes silica-alumina, silica-magnesia, silica-zirconia, silica-alumina-boric and silica-alumina-magnesia. One class of titanium-containing silicon oxide catalysts particularly suitable for regeneration by the method of the present invention is titania-on-silica, i.e. catalyst comprising titanium dioxide supported on silica (silicon dioxide). Particularly preferred are amorphous precipitated silicas and amorphous pyrogenic silicas.

In accordance with an embodiment of the present invention, the specific surface area of the siliceous-based support in between from 150-300m²/g, preferably from 120-250 m²/g, ASTM D3663 - 03(2008); a Pore Size Distribution (as determined by Nitrogen Desorption Isotherms, method ASTM: 4641-12) in the range of from 0.7-1.0 cm³/g, preferably from 0.78-0.90 cm³/g; and average pore diameter as determined by Nitrogen Isotherm, using de BJH Method (Barrett, E.P.; L.G. Joyner, P.P. Halenda (1951). "The Determination of Pore Volume and Area Distributions in Porous Substances. I. Computations form Nitrogen Isotherms". J. Am. Chem. Soc. 73), in the range of from 20-300 amgstroms, preferably from 35-165 amgstroms.

The preparation method of the catalyst may be done by a variety of techniques known in the art. For example, one of such methods include vapor-phase impregnation with a titanium tetrahalide (e.g. TiCl₄), followed by drying and then calcination at an elevated temperature. Alternatively, instead of a vapor-phase impregnation, a liquid-phase impregnation method can be used. Vapor-phase impregnation is disclosed in, e.g. EP345856, whereas liquid-phase impregnationis described in US6011162. Other methods such as calcining a mixture of inorganic siliceous solids and titanium dioxide at elevated temperature or co-gelling a mixture of a titanium salt and a silica sol may be used for the preparation of the catalyst.

The titanium-containing silicon oxide catalysts may be employed in oxidation reactions, being particularly useful for catalyzing the epoxidation of olefins with organic hydroperoxides. Olefin epoxidation processes are well known in the art.

Suitable olefins useful in epoxidation reactions include any olefin having at least one carbon-carbon double bond, and generally from 2 to 30 carbon atoms. Preferably the olefin is an acyclic alkene of from 2 to 10 carbon atoms such as ethylene, propylene, butene, pentene, hexene, heptene, octene, nonene, decene, and isomers thereof. Also preferred are olefinically unsaturated compounds substituted with a hydroxyl group or a halogen group such as allyl chloride or allyl alcohol. A particularly preferred olefin is propylene.

The organic hydroperoxide may be obtained from an hydrocarbon containing at least one secondary or tertiary carbon atom, preferably having from 3 to 20 carbon atoms, more preferably from 3 to 12 carbon atoms. The organic hydroperoxides particularly preferred are secondary alkyl hydroperoxides wherein the hydroperoxy group is on a carbon atom attached directly to an aromatic ring. Therefore, preferred hydrocarbons include isobutane, ethylbenzene, cyclohexane, isopentane, 2-methylpentane, methylcyclohexane, tetrahydronaphthalene, cumene, diethylbenzene, 3-methylpentane, and the like.

Suitable organic hydroperoxides thus include tertiary butyl hydroperoxide, tertiary amyl hydroperoxide, tertiary hexyl hydroperoxide, tertiary octyl hydroperoxide, ethyl benzene hydroperoxide, cumene hydroperoxide, cyclohexyl hydroperoxide, methyl cyclohexyl hydroperoxide, alpha-ethyl benzyl hydroperoxide, and diisopropylene benzene hydroperoxide. Particularly preferred is ethylbenzene hydroperoxide.

In accordance with a particularly preferred embodiment, the catalyst obtained in accordance with the method of the present invention is used in the co-production of propylene oxide and styrene starting from ethylbenzene. In general such process involves the steps of reacting ethylbenzene (EB) with oxygen or air to form ethylbenzene hydroperoxide (EBHP); reacting the ethylbenzene hydroperoxide (EBHP) thus obtained with propene in the presence of an epoxidation catalyst to yield propylene oxide (PO) and 1-phenyl ethanol (methyl phenyl carbinol, MPC); and dehydrating the 1-phenyl ethanol (MPC) into styrene (SM) using a suitable dehydration catalyst.

In the epoxidation reaction, the molar ratio of olefin to hydroperoxide can vary over a wide range and a molar excess of either the olefin or hydroperoxide of up to as high as 100:1 can be used. Preferably, molar ratios of olefin to hydroperoxide varying from about 50:1 to about 1:10 are satisfactory, although it is particularly preferred to employ molar ratios of olefin to hydroperoxide of about 20:1 to about 1:1.

The epoxidation reaction may be conducted in the liquid-phase in solvents or diluents that are liquid at the reaction temperature and pressure and are substantially inert to the reactants and the products produced therefrom. Generally, it may be used as a solvent the same hydrocarbon used to produce the organic hydroperoxide reactant. For example, when ethylbenzene hydroperoxide is utilized, the use of ethylbenzene as the epoxidation solvent is preferred.

Preferebly, the ethylbenzene hydroperoxide is pretreated prior to epoxidation reaction. This pretreatment includes at least a basic washing and distillation steps.

Suitable reaction temperatures vary from room temperature to 200 °C, preferably from room temperature to 160 °C, more preferably from 70 °C to 120 °C. The reaction is preferably conducted at or above atmospheric pressure. Typical pressures vary from 1 atmosphere to 100 atmospheres, preferably from 30 atmosphere to 70 atmospheres.

The epoxidation may be performed using any of the conventional reactor configurations known in the art for reacting olefin and organic hydroperoxide in the presence of an insoluble catalyst. Continuous as well as batch procedures may be used.

One preferred disposal for continous fixed bed process is a serie of reactors loaded with catalyst. The EBHP solution can be split into diferrent reactors.

Different factors, such as the identities of the olefin, organic hydroperoxide and solvent, the space velocities of the reactants, the reaction temperature and presure, and the nature and amount of impurities, can determine the exhaustion of the catalyst, and then the necesity to regenerate it.

The heterogeneous titanium-containing silicon oxide catalyst to be regenerated in accordance with the method of the present invention will normally be a catalyst which no longer has the desired activity for converting an olefin into an alkylene oxide with an organic hydroperoxide, usually propene into propylene oxide with ethylbencene hydroperoxide. Typically, it will be desirable to regenerate the catalyst when its activity is below 25 % of its activity when freshly prepared, as measured by the rate at which a given hydroperoxide reacts with a given olefin; preferably when its activity is below 20 %, more preferably between form 1-18 %, being particularly preferred when it is between from 5 - 15 %.

The used catalyst may be a catalyst which had previously been regenerated one or more times, but may also be a deactivated catalyst which deactivated for the first time and hence had not previously been subjected to a regeneration treatment.

The first organic washing (step a)) must be performed with a solvent which is inert with the catalyst active sites. Examples of suitable solvents for this first organic washing step according to the present invention include, for instance ethylbenzene, methylbencil alcohol, toluene, acetophenone, hexane, methanol, or combinations thereof. In accordance with a preferred embodiment, the solvent is ethylbenzene.

In accordance with an embodiment of the present invention, the temperature of step a) may be comprised from 20 - 90 °C, being particularly preferred from 40 - 60 °C.

Preferably, the inert solvent (e.g. ethylbenzene) to used catalyst molar ratio may be comprised from 15 : 1 to 50 : 1; more preferably from 20 : 1 to 30 : 1. The washing step a) may be preferably performed during 3-4 hours.

After the first organic washing (step a)), the used catalyst may be washed with an acid aqueous solution in order to prepare support for further Ti impregnation. The acid pretreatment of the siliceous-based support can be carried out using known methods in the art. Thus, in accordance with an embodiment of the present invention, the acid pretreatment is carried out with an aqueous solution of a strong acid; suitable acids are sulfuric acid, hydrochloric acid, phosphoric acid, nitric acid, acetic acid, and the like. Preferably, an aqueous solution of hydrochloric acid is used. The acid treatment can be carried out by immersion of the siliceous compound carrier in the acidic solution of hydrochloric acid, preferably at a temperature ranged from 20-90 °C, more preferably from 60 - 70 °C; for 2-6 hours, more preferably 3-4 hours. The acid/catalyst ratio being preferably from 1-6 ml per gram of catalyst, more preferably 2-3 ml per gram of catalyst.

At the completion of this acid treatment, the treated siliceous-based support is separated from the aqueous acid by any known method, e.g. filtration.
After the acid treatment, it is possible to perform an aqueous or alcoholic washing step, in order to minimize or completely eliminate the chloride content.

After the acid treatment it is dried at a moderate temperature ranging from 100 to 300 °C for subsequent impregnation, preferably 150 - 220 °C, being particularly preferred from 180 to 200 °C, in order to remove water. Drying may be accomplished, for example, by heating the product for several hours at a temperature of 100°C or higher.. It is highly desirable to dry the inorganic siliceous solid prior to impregnation because titanium tetrachloride reacts with water forming white fumes of hydrochloric acid. A flowing stream of a dry gas such as nitrogen must be applied to displace air, while fumes are formed upon contact with atmospheric humidity.

Once it is dried, the acid treated catalyst is impregnated with a titanium-containing agent. The impregnation step is not limited by choice of a particular titanium source, nor by any particular impregnation method. Thus, as preferred titanium source, it can be used titanium alkoxides and titanium halides, being titanium tetrachloride particularly preferred. Particularly preferred methods of impregnation include liquid-phase and vapor-phase impregation methods, which are well known in the art.

In the liquid phase supporting method impregnation may by performed by dissolving a titanium halide or titanium alkoxide (such as TiCl₄ or Ti(OiPr)₄)) in an organicsolvent and then applying the solution to the pretreated spent catalyst. Suitable solvents for this purpose are those hydrocarbons that are liquid at ambient temperatures, and are capable of solubilizing the titanium compound. Examples of suitable hydrocarbon solvents include n-hexane, n-heptane, cyclopentane, methyl pentanes, methyl cyclohexane, dimethyl hexanes, toluene, xylenes, methylene chloride, chloroform, dichloroethanes, chlorobenzene, benzyl chloride, and the like.

In accordance with another particular realisation of the liquid-phase impregnation method, the titanium compound for impregnation is Ti(OiPr)₄) and the solvent iPrOH.

In another embodiment of the present invention, the impregnation of the siliceous-based support may be performed by a vapor-phase supporting method using titanium tetrachloride as impregnating agent. Preferably, impregnation is carried out by vapor phase supporting method, wherein the vapor stream is provided by flowing a gas over liquid titanium tetrachloride. The vaporization is conducted at temperatures ranging from 100-400 °C, preferably, from 150-300 °C. Preferably, the gas is an inert gas such as nitrogen, helium, argon, carbon dioxide, and the like. The vapor stream of titanium tetrachloride is then passed over the pretreated used catalyst to complete the impregnation step.

The amount of titanium in the siliceous-based support after the impregnation step, will normally be in the range of from 0.1-5 % by weight, preferably 1-4 % wt., being particularly preferred 1.5-3.0% wt. based on the total weight of the catalyst.

Following impregnation, the impregnated solids are calcined at a temperature ranging from 400°C to 1000 °C, preferably from 550 - 950 °C, more preferably from 580 - 900 °C, being particularly preferred from 600 to 800 °C. Calcination is preferably performed in the presence of an inert gas which is substantially free of oxygen such as nitrogen, argon, neon, helium or the like or mixture thereof.

The calcination steps are preferably performed during a time ranging from 30 minutes to 18 hours, tipically from 6 to 12 hours; although short times of calcination can also possible such as from 30 minutes to 4 hours, more preferably from 45 minutes to 3 hours, particularly preferred 1-2 hours.

In accordance with a particular embodiment of the present invention, after the calcining step d), the method further comprises at least one additional step e) or f):
e) hydrolysing the regenerated catalyst; or
f) reacting the regenerated catalyst with an organic silylating agent.

Therefore, in accordance with a preferred embodiment, the regenerated catalyst is hydrolysed; preferably hydrolysis may be effected feeding water in a hot steam, of a gas carrier, usually nitrogen, at an elevated temperature, in the range of from 110 - 400 °C; more preferably from 120 - 250 °C.

After the hydrolisis step, the catalyst can be dried at a moderate temperature ranging from 100 to 300 °C preferably 150-220 °C, being particularly preferred from 180 to 200 °C , using a stream of gas or air, in order to remove water. Drying may be accomplished, for example, by heating the product for several hours at a temperature of 100°C or higher.

Alternatively, or in addition to hydrolysis, the regenerated catalyst may be reacted with an organic silylating agent. Suitably, the organic silylating agent may be selected from the group consisting of organohalosilanes, organosilylamines, organodisilazanes, and mixtures thereof. Organosilanes containing from one to three organic substituents may be utilized, including, for example, chlorotrimethylsilane, dichlorodimethyl silane, nitrotrimethylsilane, chlorotriethylsilane, chlorodimethylphenylsilane and the like. Preferred organohalosilane silylating agents include tetra-substituted silanes having from 1 to 3 halo substituents selected from chlorine, bromine, and iodine with the remainder of the substituents being methyl, ethyl, phenyl or a combination thereof.

Organodisilazanes are represented by the formula R₃Si--NH--SiR₃, wherein the R groups are independently hydrocarbyl groups (preferably, C₁-C₄ alkyl) or hydrogen. Specially preferred for use are the hexaalkyl substituted disilazanes such as, for example, hexamethyldisilazane.

Treatment with the silylating agent may be performed either in the liquid phase (i.e., where the silylating agent is applied to the catalyst as a liquid, either by itself or as a solution in a suitable solvent such as a hydrocarbon) or in the vapor phase (i.e., where the silylating agent is contacted with the catalyst in the form of a gas).

Silylation may be preceded by a separate drying step, at a temperature ranging ranging from 100 to 300 °C for subsequent impregnation, preferably 180 - 220 °C, being particularly preferred a temperature of 200 °C, in order to remove water. Drying may be accomplished, for example, by heating the product for several hours at a temperature of 100°C or higher.

In accordance with a particular embodiment of the present invention, previously to step a), the method further comprises carrying out a catalytic epoxidation of an olefin using an organic hydroperoxide and the heterogeneous titanium-containing silicon oxide catalyst. In a preferred realisation of this embodiment, the method comprises monitoring the catalyst deactivation during said catalytic epoxidation reaction, and stopping the said reaction when the activity of the catalyst is below 25 %of its activity when freshly prepared, as measured by the rate at which a given hydroperoxide reacts with a given olefin; preferably when its activity is below 20 %, more preferably between form 1-18 %, being particularly preferred when it is between from 5 - 15 %. Once stopped the reaction, the deactivated catalyst is regenerated in accordance with the process as previously described.

Tipically, carrying out the catalytic epoxidation reaction includes mixing the reactants (organic hydroperoxide solution and the olefin) in the reactor in presence of a catalyst bed to obtain the epoxide.

Stopping the reaction includes stopping the flow of reactants through the catalyst bed, and optionally remove the catalyst from the reactor.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Example 1.

A titania on silica heterogeneous catalyst was manufactured following the procedure described in EP345856, including the steps of Ti impregnation, calcination, hydrolysis and silanization.

Briefly, dried silica pellets were impregnated with a stream of gaseous TiCl₄ at 200 °C. Calcining the impregnated silica at 600 °C during 6 hours.
Hydrolysis at 200 °C and silanization with a solution of hexamethyldisilazane in toluene.

The fresh catalyst contains 1.6 % wt. of Ti and was used in the epoxidation of propylene with EBHP in a fixed bed reactor during 1260 hours. The used catalyst (CATALYST 1) was removed from the reactor and was dried with air. The activity of the used catalyst was 20% of the fresh catalyst.

This used catalyst was regenerated by different methods:

### Comparative Example 1:

Used catalyst was washed with ethylbenzene. The liquid solid ratio was 30, the washing temperature was 60°C and the washing time 4 hours (CATALYST 2).

### Example 1:

Used catalyst was washed with ethylbenzene. The liquid-solid ratio was 30, the washing temperature was 60°C and the washing time 4 hours. Following, the catalyst was washed with acid hydrochloric (4N) at 70 °C for 4 hours. The acid/catalyst ratio was 3 ml per gram of catalyst. The acid treated catalyst was dried and impregnated with TiCl₄. Calcining over 450 ° C. Hydrolysis with water vapor and Silanization. (CATALYST 3)

### Comparative Example 2:

Used catalyst was treated as in EP1251960, although previously to the decoking treatment it was washed with ethylbenzene. Accordingly, used catalyst was washed with ethylbenzene. The liquid solid ratio was 30, the washing temperature was 60°C and the washing time 4 hours. Washed catalyst was subjected to a decoking treatment at 600°C during 8 hours. Calcinated catalyst was then washed with acid hydrochloric (4N) at 70 °C for 4 hours. The acid/catalyst ratio was 3 ml per gram of catalyst. The used catalyst was dried and impregnated with TiCl₄. Calcining over 450 ° C. Hydrolysis with water vapor and Silanization. (CATALYST 4)

### Comparative Example 3:

Used catalyst was calcinated at 600°C during 8 hours as in EP1539346. (CATALYST 5).

### Example 3: Performance of the catalysts

### General method:

The content of % Ti wt. in the fresh catalyst was 1.6%w Ti, while the spent catalyst after 1260h of operation contained 1.4%w Ti. The evolution of Ti content after treatments carried out was as follows:

| | CATALYST 3 | CATALYST 4 |
|---|---|---|
| After Acid treatment | 0,97% | 1,03% |
| Regenerated catalyst | 2,51% | 2,67% |

None of the treatments prior to Ti reloading completely removed Ti from the silica support and the regenerated catalyst contain higher titanium than fresh catalyst.

To evaluate the performance of the catalysts, batch epoxidations of propylene with ethylbenzene hydroperoxide were carried out. The procedure is described as follows:
The catalyst performance was tested in a batch stirred tank reactor. The reactor volume was 1 liter and the catalyst is put on a basket inside the reactor. The catalyst loading was 3g.

The epoxidation of propene was carried out with a solution of ethylbenzene hydroperoxide. The ethylbenzene hydroperoxide contains 35%w of hydroperoxide in ethylbenzene and it was made by oxidation of ethylbenzene with air. 150 g of ethylbenzene hydroperoxide solution and 250 g of propylene were introduced in the reactor. The reactor was heated at 100°C. Once the reactor reached the set temperature (100°C) several samples (0, 90, 150 and 300 min) were taken from the reactor in order to analyze the composition by GC and HPLC. The total epoxidation time was 300 min.

### Activity results:

Batch epoxidation tests were repeated three days with fresh feed and same catalyst in order to check the lost in activity and selectivity.

The results are presented in the following table:

| | **FRESH CATALYST** | **CATALYST 2** | **CATALYST 3** | **CATALYST 4** | **CATALYST 5** |
|---|---|---|---|---|---|
| **Test 1** | | | | | |
| k' (l/ h g) | 0,257 | 0,047 | 0,179 | 0,179 | 0,110 |
| Activity (%) | 100 | 18,6 | 70 | 70 | 43 |
| S ACP/EBHP | 3,4 | 8,1 | 3,7 | 4,7 | 10,0 |

| **Test 2** | | | | | |
|---|---|---|---|---|---|
| k' (l/ h g) | 0,250 | 0,048 | 0,170 | 0,135 | 0,092 |
| Activity (%) | 97 | 18,7 | 66 | 52 | 36 |
| S ACP/EBHP | 3,7 | 6,1 | 3,7 | 4,2 | 11,6 |

| **Test 3** | | | | | |
|---|---|---|---|---|---|
| k' (l/ h g) | 0,197 | 0,0474 | 0,149 | 0,119 | 0,088 |
| Activity (%) | 76,7 | 18,4 | 58 | 46 | 34 |
| S ACP/EBHP | 4,6 | 5,7 | 2,9 | 4,1 | 12 |

| | | | | | |
|---|---|---|---|---|---|
| Notes: 1. **k':** pseudo-kinetic rate constant, considering propylene concentration constant during epoxidation reaction 2. **Activity:** ratio between k' of a sent or regenerated catalyst and k' of fresh catalyst 3. **S ACP/EBHP:** molar selectivity of ACP | | | | | |

The EB washing didn't increase catalyst activity in CATALYST 2, whereas CATALYST 3 and 4 showed an important increase in activity but CATALYST 3 yielded better selectivity and a slow decrease in activity. Finally, CATALYST 5 improved activity but far away from CATALYST 3 and 4 and with poor selectivity.

### REFERENCES CITED IN THE APPLICATION

1. US4367342
2. EP345856.
3. US4367342
4. EP345856
5. US6011162
6. WO9828072
7. US5798313
8. EP1251960
9. EP1539346
10.ASTM Standard D3663 - 03(2008)" Test Method for Surface Area of Catalysts and Catalyst Carriers " ASTM International, West Conshohocken, PA,
11.ASTM: 4641-12, "Standard Practice for Calculation of Pore Size Distributions of Catalysts and Catalyst Carriers from Nitrogen Desorption Isotherms",ASTM International, West Conshohocken, PA

## Claims

1. A process for regenerating a heterogeneous titanium-containing silicon oxide catalyst comprising the steps of:
a) contacting the heterogeneous titanium-containing silicon oxide catalyst to be regenerated with a solvent which is inert with the catalyst active sites selected from the group consisting of ethylbenzene, methylbencil alcohol, toluene, acetophenone, hexane, methanol, and combinations thereof, at a temperature ranging of from 20-90 °C, during 2-6 hours;
b) washing the catalyst obtained in step a) with an acid aqueous solution at a temperature ranging from 20-90 °C for 2 - 6 hours;
c) impregnating the washed catalyst thus obtained with a titanium-containing impregnating agent; and
d) calcinating the impregnated catalyst at a temperature ranging from 400 - 900 °C to obtain a regenerated catalyst.

2. The process of claim 1, wherein the solvent of step (a) is ethylbenzene.

3. The process according to any of claims 1-2, wherein the temperature of step a) is from 40 - 60 °C.

4. The process according to any of claims 1-3, wherein step a) is performed at a ethylbenzene : catalyst molar ratio comprised from 15 : 1 to 50 : 1.

5. The process according to any of claims 1-4, wherein the acid aqueous solution used in step b) is an aqueous solution of hydrochloric acid.

6. The process according to any of the claims 1-5, wherein the titanium-impregnating agent is a liquid impregnating agent or a gaseous impregnating agent.

7. The process according to claim 6, wherein the titanium-impregnating agent is gaseous titanium tetrachloride.

8. The process according to any of claims 1-7, which after the calcining step d) further comprises at least one additional step e) or f):
e) hydrolysing the regenerated catalyst; or
f) reacting the regenerated catalyst with an organic silylating agent.

9. The process according to claim 8, wherein the organic silylating agent is selected from the group consisting of organohalosilanes, organosilylamines, organodisilazanes, and mixtures thereof.

10. The process according to claim 9, wherein the organic silylating agent is an organodisilazane.

11. A process for the preparation of an alkylene oxide comprising
a) carrying out the process of regenerating the heterogeneous titanium-containing silicon oxide catalyst of any of the claims 1-10; and
b) reacting an olefin with an organic hydroperoxide in the presence of the regenerated heterogeneous titanium-containing silicon oxide catalyst of step a).

12. The process according to claim 11, further comprising a previous step of carrying out a catalytic epoxidation of an olefin using an organic hydroperoxide and a heterogeneous titanium-containing silicon oxide catalyst.
